# EUROPÄISCHE PATENTSCHRIFT

(11) **EP 0 902 658 B1**
(45) Veröffentlichungstag und Bekanntmachung des Hinweises auf die Patenterteilung: **01.08.2001**
(21) Anmeldenummer: 97923031.5
(22) Anmeldetag: 09.05.1997
(51) Int. Cl.: A61B 17/72, A61F 2/30

(54) **Vorrichtung zum Transport eines Knochensegments zur Überbrückung eines Knochendefekts**
Bone segement conveying device for bridging a bone damage
Dispositif pour transporter un segment osseux destiné à réparer une lésion osseuse

(30) Priorität: 09.05.1996 DE 19618552
(43) Veröffentlichungstag der Anmeldung: 24.03.1999
(73) Patentinhaber: Horas, Uwe, 60433 Frankfurt am Main (DE)
(72) Erfinder: HORAS, Uwe Dr., 60433 Frankfurt am Main (DE); ERBEN, Markus, 65795 Hattersheim (DE)
(74) Vertreter: Funck-Hartherz, Anna-Eleonore, Dipl.-Phys.
(86) Internationale Anmeldenummer: EP9702370
(87) Internationale Veröffentlichungsnummer: WO9742896

(56) Entgegenhaltungen:
- EP-A- 0 548 535
- WO-A-94/18897
- WO-A-95/24870
- US-A- 5 352 227

## Beschreibung

Die Erfindung betrifft eine Vorrichtung zum Transport eines Knochensegments zur Überbrückung eines Knochendefektes von sich an ein Gelenk anschließenden Röhrenknochen.

Überbrückungen von Knochendefekten an langen Röhrenknochen sind in der unfallchirurgischen und orthopädischen Praxis eine häufige Behandlungsnotwendigkeit. Hierbei geht es um die Schaffung neuen Knochengewebes über das Verschieben eines erhaltenen Knochensegmentes am langen Röhrenknochen im wesentlichen durch die osteogene Potenz der Knochenhaut.

Die Segmentverschiebung ist ein erprobtes und seit langem bekanntes Verfahren. Die für den Segmenttransport erforderliche Kraft wir derzeit in der Regel extracorporal über sogenannte externe Fixateur Systeme (äußerer Spanner) oder nach der neuesten Entwicklung voll implantiert über einen intercorporell liegenden Elektromotor und ein intrameduläres Implantat (Marknagel) erzeugt. Ein weiteres Verfahren, allerdings ausschließlich zur Verlängerung von Röhrenknochen verwendbar, benutzt für seine Krafteinleitung ein Rotationsprinzip (Rohr im Rohr) in Verbindung mit einer Ratsche. Ferner ist eine vollständig implantierte Vorrichtung aus der WO 95/24 870 bekannt geworden. Diese sieht im Modullarkanal ein den Defekt überbrückendes Mantelrohr vor, an dessen Ende ein weiteres Rohr mit begrenzter Drehbarkeit angeordnet ist, wobei diese Rohre proximal und distal jenseits des Defekts an gesunden Knochenteilen fixiert sind. Diese Rohre enthalten mit dem Knochensegment verbundere Mittel, die bei Einwirken einer äußeren Drehkraft im Bereich des distalen Knochens eine vorgegebene Verschiebung des Knochensegments erreichen. Diese Drehung muß von einer medizinisch ausgebildeten Person erfolgen und des weiteren hat diese Methode den Nachteil, daß es in der letzten Phase der Verschiebung der Segmentanlage an den gesunden Knochen zu Schwierigkeiten kommt, dadurch die Drehung des distalen Knochens fortlaufende Verletzungen der Knochenhaut erfolgen, die einen knöchernen Anbau verhindern. Alle diese bekannten Techniken bringen zum Teil erhebliche Nachteile für den Patienten wie Infektionsrisiko bei Verwendung äußerer Spanner, unkomfortable Handhabung und lange stationäre Behandlungsdauer mit sich. Darüber hinaus entstehen, wie im Falle des Implantates mit nicht wieder verwendbarem Elektromotor, unverhältnismäßig hohe Kosten.

Aufgabe der Erfindung ist es, eine Vorrichtung zum Transport eines Knochensegmentes zu entwickeln, die vollständig implantiert ist, autoinduktiv, d. h. ohne fremde Hilfe, vom Patienten selbst betrieben werden kann, und die obengenannten Nachteile vermeidet.

Die Erfindung löst die Aufgabe durch eine Vorrichtung, wie sie im kennzeichnenden Teil des Anspruchs 1 beschrieben ist. Das grundsätzlich Neue an der erfindungsgemäßen Vorrichtung ist die Einbeziehung des an den beschädigten Röhrenknochen angrenzenden Gelenks. Zum Antrieb des Knochensegments wird die translatorische Bewegung eines Zugseiles benutzt, das mit einem Ende am durch das Gelenk mit dem defekten Knochen beweglich verbundenen gesunden Knochen streckseitig mit Abstand zum anatomisch-physiologischen Gelenkdrehpunkt verankert ist, die Gelenkköpfe und das Gelenk außerhalb des Gelenkdrehpunktes überspannt und in der Markhöhle des defekten Knochens im Inneren eines Marknagels geführt ist und mittelbar an dem zu bewegenden Knochensegment angreift. Der Seilzug kann vom Patienten autoinduktiv erzeugt werden. Er benötigt keine systemfremde Energiequelle und kommt mit einer einfachen wirtschaftlichen Vorrichtung aus. Der Patient kann den Verfahrensablauf ohne ärztliche Hilfe selbst steuern. Eine passive Bewegung ist ebenfalls möglich, sofern der Patient die aktive Bewegung selbst nicht ausführen kann.

Die Vorrichtung ist voll implantiert und umfaßt keine extrascorporellen Komponenten. Die Vorrichtung kann am Kniegelenk und am Ellenbogengelenk angewendet werden. Voraussetzung für seine Anwendung ist eine ausreichende Gelenkbeweglichkeit hinsichtlich Beugung / Streckung.

Eine erfindungsgemäße Vorrichtung weist ein Zugseil oder dgl. Zugelement auf, das mit seinem einen Ende an dem gesunden Knochen des Gelenkes durch eine Verankerung befestigbar und mit seinem anderen Ende über einen die Transportstrecke vergebenden Antrieb derart mit dem Knochensegment verbindbar ist, daß durch Beugebewegung des gesunden Knochens relativ zu dem defekten Knochen eine Zugkraft und eine translatorische Bewegung von der Verankerung auf das Knochensegment übertragbar ist. Weiterhin umfaßt die erfindungsgemäße Vorrichtung einen Antrieb, der die translatorische Bewegung des Zugseils auf neuartige Weise in eine achsiale Segmentverschiebung umsetzt.

Weitere Einzelheiten der Erfindung sind der beigefügten Zeichnung zu entnehmen.

Es zeigen:
- Fig. 1: einen Schnitt durch eine erfindungsgemäße Vorrichtung in Knochenlängsache am Beispiel eines defekten Oberschenkelknochens.
- Fig. 2 und 3: zwei um 90° gedrehte Schnitte eines erfindungsgemäßen Antriebes in Längsachse des Marknagels.

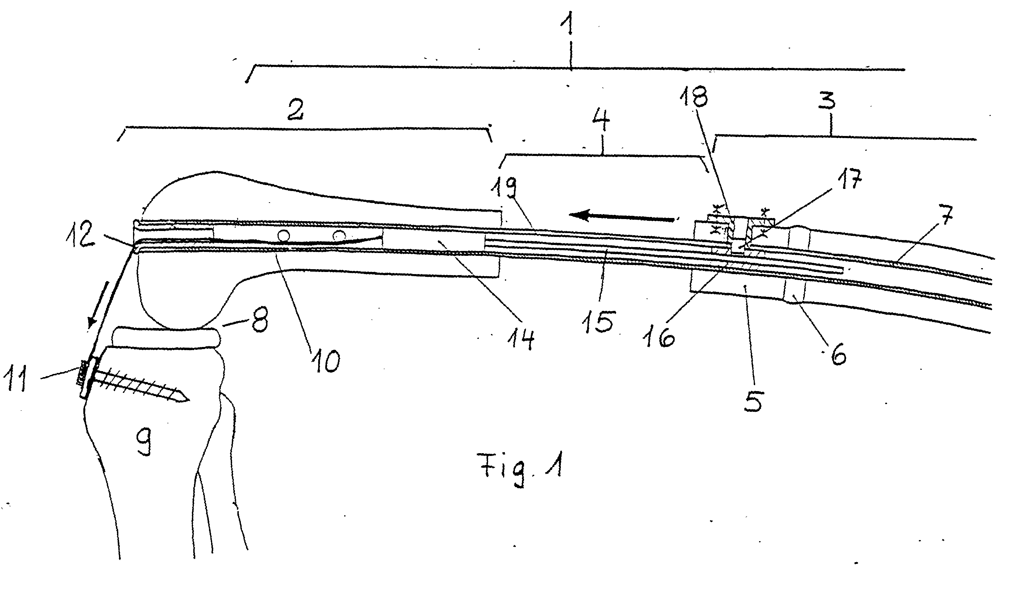

Im Beispiel nach Fig. 1 werden durch einen intramedullär eingetriebenen Marknagel 7 die erhaltenen körperfernen und körpernahen Knochenteile 2, 3 eines defekten Oberschenkelknochens 1 gestützt und im Abstand des Defektes 4 zueinander in Position gehalten. Ein erhaltenes Knochensegment 5 ist vom proximalen (körpernahen) Knochenteil 3 osteotomiert (mit Meißel unter Erhalt der Knochenhaut abgespalten) und soll zur Überbrückung des Defektes 4 in Richtung auf das distale (körperferne,) Knochenteil 2 transportiert werden. Bei wachstumsgerechter Einstellung der Transportgeschwindigkeit des Knochensegementes 5 wird vom Kallus 6 der sich stetig erweiternde Raum zwischen Segment 5 und proximalem Knochenteil 3 durch neu gebldetes Knochengewebe gefüllt, so saß bei abgeschlossenem Transport des Knochensegmentes 5 der Defekt überbrückt ist. Für den Transport wird das Knochensegment 5 in geeigneter Weise mit einem Antrieb 14 verbunden, vorzugsweise mit einer Knochenhülse 18 mittels einer Segementschraube 17 auf eine Spindelmutter 16 geschraubt. In der Spindelmutter 16 ist eine Gewindestange 15 mit ihrem Gewinde eingedreht. Die Gewindestange 15 ist von dem Antrieb 14 antreibbar, d. h. drehbar, und transportiert durch ihre Drehung in der Mutter 16 das Knochensegment 5 über den Marknagel 7 zum Anschluß an den distalen Knochenteil 2, wobei das Knochensegment 5 durch die Schraube 17 in einen Schlitz 19 des Marknagels 7 geführt wird. Die Verbindung zwischen dem Knochensegment 5 und dem Antrieb 14 im vorliegenden Beispiel die Gewindestange 15, verläuft dabei innerhalb des Marknagels 7. Der Antrieb 14 bewirkt hierbei, daß bei Beugung des Gelenkes zur Anregung der osteogenen Aktivität der Knochenhaut die notwendige anatomisch optimierte Zugrichtung und zeitlich biologisch optimierte Zugkraft auf das Segment ausgeübt wird.

Im Gelenk 8, im Beispiel dem Kniegelenk, sind der defekte Oberschenkelknochen 1 und der gesunde Unterschenkelknochen 9 miteinander schwenkbar verbunden. Am Gelenkkopf de gesunden Knochens 9 ist ein Zugseil 10 in einer Verankerung 11 befestigt. Das Zugseil 10 ist von der Verankerung 11 exzentrisch zum anatomischphysilogischen Drehpunkt des Gelenkes 8 streckseitig über das Gelenk 8 durch den ausgerundeten Mund einer in das gelenkseitige Ende des distalen Knochenteils 2 eingesetzten Tülle 12 und einem Durchgangskanal des sich anschließenden Marknagels 7 zugkraftschlüssig in den Antrieb 14 eingeführt. Durch Schwenkung des gesunden Knochens 9 um den Gelenkdrehpunkt im Gegenuhrzeigersinn relativ zu dem defekten Knochen 1 wird das Zugseil 10 in Pfeilrichtung aus dem Marknagel 7 herausgezogen und eine gleichgerichtete translatorische Seilbewegung und Zugkraft erzeugt. Seilbewegung und Zugkraft werden mittels des Antriebs 14 in eine axiale Segmentverschiebung umgesetzt und damit das Knochensegment 5 in Richtung des distalen Knochenteils 2 transportiert, wodurch eine Schließung des Defektes zum distalen Knochenteil 2 hin erreicht wird. In der dargestellten Ausführung werden die Seilbewegung und die Zugkraft in dem analog einem mechanisch angetriebenen Spielkreisel wirkenden Antrieb 14 in eine die Mutter 16 und damit das Knochensegment 5 in Richtung des distalen Knochenteils 2 transportierenden Drehbewegung der Gewindestange 15 umgewandelt.

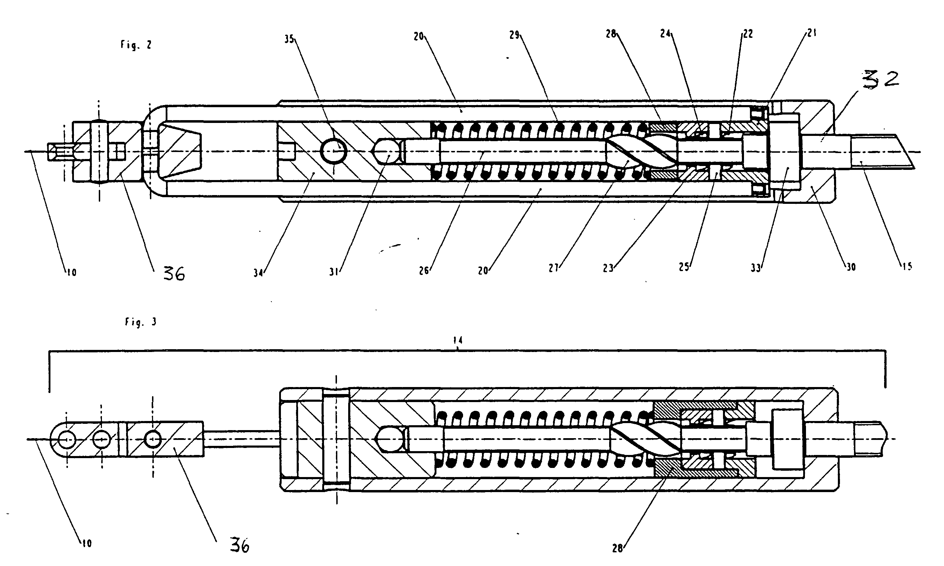

Ein Ausführungsbeispiel eines solchen Antriebes 14 ist in de Figuren 2 und 3 dargestellt. Der Antrieb 14 weist ein zylindrisches Gehäuse 30 auf, in dem eine Spindel 26 durch ein zu Montagezwecken ausbaubares, im Gehäuse 30 durch einen Bolzen 35 fixiertes rückwärtiges Lager 31 und ein vorderes Lager 32 gelagert und durch einen Bund 33 gegen Verschiebung in ihrer Achse gesichert ist. Auf der Spindel 26 sind in der Reihenfolge von vorn (dem dem Gelenk 8 abgekehrten Ende des Antiebes 14) nach hinten beweglich und drehbar ein vorderes Kupplungsteil 21, ein rückwärtiges Kupplungsteil 23, eine Hülse 28 und eine Schraubenfeder 29 angeordnet. Die in Richtung auf das Gelenk 8 wirkende Zugkraft und translatorische Bewegung aus dem Zugseil 10 werden in einem Anschlußsegment 36 in den Antrieb eingeleitet. Zugstangen 20 übertragen die translatorische Bewegung auf die vordere Kupplungshälfte 21. Sie wird um einen Abstand 25 der Kupplungshäften 21, 23 zurückgezogen und gegen die rückwärtige Kupplungshälfte 23 gepreßt, wobei an beiden Kupplungshälften 21, 23 angeordnete Zähne 22 und 24 zum Eingriff kommen. Die Kupplungshälfte 23 der Kupplung ist gleichzeitig Mutter für das Gewinde 27 auf der Spindel 26. Die Charakteristik des Gewindes 27 ist so gewählt, daß bei Einwirken des Seilzuges die Mutter sich leicht auf das Gewinde 27 der Spindel 26 aufschieben läßt und dabei die Spindel 26 zur Rotation bringt, die dann über ein langes Gewindeende 15 zur Mutter 16 am Knochensegment 5 übertragen wird und den Transport des Segmentes über den Defekt 4 bewirkt.

Das Gewinde 27 der Spindel 26 weist eine von der gewählten Gewindesteigung abhängige Länge auf. Die Länge des Gewindes 27 bestimmt sich dadurch, daß die von ihm bei der translatorischen Bewegung der Kupplungshälfte 23 erzeugte Zahl der Spindeldrehungen in der Mutter 16 eine nach medizinischen Erfordernissen bestimmte begrenzte Transportstrecke des Segementes 5, z. B. 1 mm. Zur Erreichung der gewünschten Transportstrecke ist eine genaue Abstimmung der Gewinde von Spindel 26 und Mutter 16 erforderlich.

Über die als Mutter 23 wirkende Kupplungshilfe ist die becherförmige Hülse 28 frei drehbar und ohne Verbund gestülpt. Sie wird mit der Kupplungshälfte 21 durch die Zugstangen 20 zurückbewegt und spannt damit die Schraubenfeder 29. Zur Erreichung der benötigten Transportstrecke des Segmentes 5 bzw. der dafür erforderlichen Drehungen in der Mutter 16 muß die rückwärtige Kupplungshälfte 23 (= Mutter für das Spindelgewinde 27) voll, d. h. mindestens bis hinter das Gewinde 27 auf der Spindel 26 zurückbewegt werden. Ein weiterer Rückzug der Mutter 23 bleibt wirkungslos, da die Spindel 26 dort gewindefrei ist. Dieser Teil der Spindel 26 bildet den Spielraum der Zugweges des Seiles 10 und dient dem Ausgleich (der Pufferung) eines ungeregelten Seilzuges, die bei der ungeregelten Gelenkbewegung des Patienten im Gelenk 8 unvermeidlich ist. Mit Beendigung des Zuges im Zugseil 10 drückt die Feder 29 über die Hülse 28 die Kupplungshälfte 21 und - nachdem die Kupplungshälfte 23 entkuppelt ist - auch diese wieder in die Ausgangsstellung zurück. Bei der Rückstellung durch den Federdruck wird durch entsprechende Ausbildung der Hülse 28 zuerst das vordere Kupplungsteil 21 auf den Abstand 25 abgedrückt und dadurch das Kupplungsteil 23 und die Spindel 26 entkuppelt. Der Antrieb 14 ist für einen erneuten Transport des Knochensegmentes 5 bereit.

## Patentansprüche

1. Vorrichtung zur Verschiebung eines Knochensegments (5) zur Überbrückung eines Knochendefektes (4) an einem an ein Knie- bzw. Ellenbogengelenk (8) angelenkten defekten Knochen (1) mit einem hohlen im Medullar-Kanal befindlichen Marknagel (7) zum Aufabstandhalten und Fixieren der den Defekt begrenzenden Knochenteile (2,3) und zur Fixierung und Translation des mit einem Knochenteil (3) des defekten Knochens (1) durch Kalus (6) verbundenen Knochensegments (5),
**dadurch gekennzeichnet,**
daß ein Zugseil (10) oder dergl. Zugelement sowie ein Antrieb (14) vorgesehen sind, wobei das Zugseil (10) mit einem Ende am durch das Gelenk (8) mit dem defekten Knochen (1) beweglich verbundenen gesunden Knochen (9) streckseitig mit Abstand zum anatomisch-physiologischen Drehpunkt des Gelenks verankerbar ist, sich von dieser Verankerung (11) exzentrisch zum Drehpunkt des Gelenks (8) über das Gelenk durch eine Öffnung und einen Durchgangskanal des Marknagels (7) im gelenknahen Knochenteil (2) des defekten Knochens (1) intramedullär erstreckt, mit seinem anderen Ende an dem im Marknagel (7) fixierbaren und im Bereich des gelenkfernen Endes des gelenknahen Knochenteils (2) angeordneten Antrieb (14) angreift und durch Beugung des gesunden Knochens (9) im Gelenk (8) eine translatorische Bewegung erfährt und in den Antrieb (14) weitergibt, und wobei der Antrieb (14) so aufgebaut ist, daß er die Transportstrecke des Knochensegmentes (5) - das in der nach medizinischen Erfordernissen bestimmte begrenzte Weg des Knochensegments (5) bei einer Beugebewegung des gesunden Knochens (9) - vorgibt, und die translatorische Bewegung des Zugseils (10) in eine das Knochensegment (5) in Richtung auf eine Schließung des Knochendefektes antreibende Bewegung umwandelt.

2. Vorrichtung nach Anspruch 1,
**dadurch gekennzeichnet,**
daß am gelenkseitigen Ende des Marknagels (7) eine die Einleitung des Zugseils (10) ausrundende Tülle (12) angeordnet ist.

3. Vorrichtung nach einem oder mehreren der vorhergehenden Ansprüche,
**dadurch gekennzeichnet,**
daß der Antrieb (14) über eine Gewindestange (15) und eine Mutter (16) mit dem Knochensegment (5) verbunden ist.

4. Vorrichtung nach Anspruch 3,
**dadurch gekennzeichnet,**
daß das Knochensegment (5) auf der Mutter (16) der Gewindestange (15) mit einer in einer Knochenhülse (18) aufgenommenen Segmentschraube (17) fixierbar ist.

5. Vorrichtung nach Anspruch 4,
**dadurch gekennzeichnet,**
daß die Mutter (16) mit der Segmentschraube (17) in einem über den Defekt (4) des Knochens (1) sich erstreckenden Schlitz (19) des Marknagels (17) geführt ist.

6. Vorrichtung nach einem der Ansprüche 3 bis 5,
**dadurch gekennzeichnet,**
daß der Antrieb (14) die folgenden Elemente aufweist:
ein innen im Marknagel (9) - z. B. durch Gewinde - fixierbares zylindrisches Gehäuse (30);
eine im Gehäuse (30) über ein vorderes Lager (32) und ein rückwärtiges lösbares Lagerteil (31) drehbar und in ihrer Längsachse unverschiebbar gelagerte Spindel (26) mit einem Gewindeteil (27) und einem durch das vordere Lager (32) aus dem Gehäuse (30) herausstehenden und die Gewindestange (15) bildenden Abschnitt;
eine auf der Spindel (26) drehbar und verschiebbar angeordnete Kupplung mit einem vorderen Kupplungsteil (21) und einem im entkuppelten Zustand mit Abstand (25) auf der Spindel (26) verschobenen rückwärtigen Kupplungsteil (23), das zugleich als Mutter für den Gewindeteil (27) der Spindel (26) ausgebildet ist;
eine Hülse (28), die mit ihrer Wand das rückwärtige Kupplungsteil (23) gleitend übergreift und mit dem Hülsenrand gegen das vordere Kupplungsteil (21) drückt;
eine z. B. als Schraubenfeder (29) ausgebildete Rückstellfeder, die sich einerseits gegen das Gehäuse (30), andererseits gegen die Hülse (28) abstützt; sowie
ein Anschlußelement (36), an welches das Zugseil (10) anschließbar ist, und von dem an dem Anschlußelement (36) angreifende, im Gehäuse (30) geführte Zugstangen (20) die translatorische Bewegung des Zugseils (10) auf die vordere Kupplungshälfte (21) übertragen.

## Claims

1. Device for displacing a bone segment (5) and spanning a bone defect (4) in a knee-or elbow joint (8) related bone (1). A bone marrow pin (7) located in a hollowing of the medulla canal secures both end sections (2,3) of the defective bone (1) at a fixed distance, and is also used for the securing and translation of the bone segment (5), which is connected by a callus (6) to the bone section (3) of the defective bone (1).
**Characterised by,**
the provision of a traction cord (10) or a similar traction element as well as a drive unit (14) through which the traction cord (10), with one end attached to an anchor (11) on the mobile healthy bone (9) on the opposite side of the joint (8) of the defective bone (1), on the outer side distanced from the anatomical-physiological centre of rotation. The traction cord (10) is stretched from the anchor (11) over the joint through an opening and intra-medulla through a transitory canal in the bone marrow pin (7) in the bone part (2) of the defective bone (1), and attached to the drive unit (14) located in the bone marrow pin at the end furthest from the joint (8) of the joint nearest bone part (2) region. A bending of the limb (1,9) at the joint (8) will result in a translational motion of the drive unit (14) end of the traction cord (10), which is then passed on into the drive unit (14) itself through which the drive unit (14) being constructed in such a manner that it establishes the transport path of the bone segment (5) (in accordance with medical requirements that determine the limited path of the bone segment (5) with a bending of the limb (1,9) at the joint (8)) and conveys the translational movement of the traction cord (10) into a driving motion of the bone segment (5) in the direction that leads to the closing of the bone defect.

2. Device in accordance with claim 1,
**Characterised by,**
a belled-out bush (12) that is located in the joint end of the bone marrow pin (10) to guide the traction cord (7) into the bone marrow pin (7).

3. Device in accordance with one or more of the previous claims,
**Characterised by,**
the linking of the drive unit (14) with the bone segment (5) via a threaded rod (15) and a female screw (16).

4. Device in accordance with claim 3,
**Characterised by,**
the securing of the bone segment (5) to the female screw (16) of the threaded rod (15) by means of a segment screw (17) located in a bone bush (18).

5. Device in accordance with claim 4,
**Characterised by,**
the channelling of the female screw (16) together with the segment screw (17) along a slot which stretches across the length of the defect (4) of the bone (1), in the bone marrow pin (19).

6. Device in accordance with one of the claims 3 to 5,
**Characterised by,**
the following contents of the drive unit (14):
A cylindrical housing (30) within the bone marrow pin (9) is securable for example by means of a thread;
A part supported within the housing (30) by an anterior bearing (32) and a loose rearward bearing part (31) is rotatable and together with a screw part (27) and the anterior bearing (32) that projects out of the housing (30) and connects with the threaded rod (15), is rigrd along its longitudinal axle situated spindle (26).
A coupling that is rotatable and traversable on the spindle (26) and is comprised of an anterior coupling part (21) and with a displacement (25) along the spindle (26), a rearward coupling part (23) that is also shaped as a female screw for the screw part (27) of the spindle (26).
A sleeve (28), that slides over the rearward coupling part (23) and pushes with its rim against the anterior coupling part (21).
A readjust-spring for example in the form of a coil-spring, is supported at one end by the housing (30) and at the other end by the sleeve (28); along with
a connecting joint (36) to which the traction cord (10) is connectable, and from which connecting rods (20) (which the connecting joint (36) pulls on, and are guided within the housing (30)) transmit the movement of the traction cord (10) to the anterior coupling half (21).

## Revendications

1. Dispositif pour le déplacement d'un segment osseux (5) dans le but de surmonter une région osseuse défectueuse(4) d'un os défailli (1) relié de façon articulée à l'articulation du genou ou du coude (8) grâce à un clou médullaire (7) situé dans un canal médullaire creux et destiné à maintenir un écart entre les parties osseuses limitrophes et à fixer les parties osseuses limitrophes (2,3) de la région défectueuse, dans le but d'obtenir une fixation et une translation du segment osseux (5) relié lui-même à une partie osseuse (3) de l'os endommagé (1) par un cal (6),
**caractérisé par le fait**
qu'il comprend un tendeur (10) ou un élément similaire propre à exercer une traction, ainsi qu'un élément moteur (14), le tendeur (10) étant ancré par une de ses extrémités dans la partie sous traction de l'os sain (9) relié lui même, de façon mobile et avec écart par rapport au pivot anatomique-physiologique de l'articulation (8), avec l'os endommagé (1), le tendeur (10) s'étendant à partir de l'ancrage, de façon excentrique par rapport au pivot de l'articulation (8), et passant, par voie intramédullaire, par un orifice et par un canal du clou médullaire (7) dans la partie osseuse (2) de l'os endommagé (1) près de l'articulation, et
qu'il s'engrène, par son autre extrémité, avec l'élément moteur (14) qui peut être fixé dans le clou médullaire (7) et qui est placé au bout distant de l'articulation dans la partie osseuse (2) qui elle-même est située près de l'articulation, et
que le dit élément moteur (14) effectue, par fléchissement de l'os sain (9), une translation dans l'articulation (8), cette translation étant transmise à l'élément moteur (14) - à noter ici que l'élément moteur (14) est construit de façon à prédéterminer le parcours de translation du segment osseux (5), veut dire le parcours du segment osseux (5) à la suite d'un fléchissement de l'os sain (9), qui est déterminé et limité par les exigences médicales, ainsi transformant la translation du tendeur (10) en un mouvement qui pousse le segment osseux (5) à refermer la partie défaillie de l'os.

2. Dispositif selon revendication 1,
**caractérisé par le fait**
qu'un orifice en forme de fourreau au rebord arrondi (12) permet l'introduction du tendeur (10) à l'extrémité du clou médullaire (7) positionné du côté de l'articulation.

3. Dispositif selon une ou plusieurs des revendications précédentes,
**caractérisé par le fait**
que l'élément moteur (14) est relié avec le segment osseux (5) par une broche filetée (15) et un écrou (16).

4. Dispositif selon la revendication 3,
**caractérisé par le fait** que le segment osseux (5) peut être fixé sur l'écrou (16) de la broche filstée (15) par une vis à segments (17) placée dans un manchon osseux (18).

5. Dispositif selon la revendication 4,
**caractérisé par le fait**
que l'écrou (16) avec la vis à segments (17) est guidé dans une rainure (19) du clou médullaire (17) qui s'étend sur la région défectueuse (4) de l'os (1).

6. Dispositif selon les revendications 3 à 5,
**caractérisé par le fait**
que l'élément moteur (14) comprend les éléments suivants:
un boîtier cylindrique (30) fixable à l'intérieur du clou médullaire (9), par exemple par filetage,
une broche rotative (26) à l'intérieur du boltier (30), tournant dans un palier avant (32) et un palier arrière détachable , broche qui ne permet le déplacement sur son axe longitudinal et qui est munie d'une pièce filetée (27) et d'une partie débordant du boîtier (30) par le palier avant (32) et formant la tige filetée ( 15), et
une pièce de jonction rotative et déplaçable placée sur la broche (26) et comprenant une partie de jonction avant (21) et une partie de jonction arrière (23) qui, à l'état découplé, est déplacée sur la broche filetée (26) à la distance (25), servant en même temps d'écrou pour la partie filetée (27) de la broche (26), et
un manchon (28) dont la paroi embrasse la partie de jonction arrière (23) de façon glissante et l'appuie, avec son rebord, contre la partie de jonction avant (21), et
un ressort de rappel (29) sous la forme, par exemple, de ressort hélicoïdal qui s'appuie d'un côté contre le boîtier (30) et d'autre côté contre le manchon (28), ainsi
qu'un élément de raccordement (36) auquel on peut raccorder le tendeur (10), et duquel des tiges de traction (20) placées dans le boîtier (30) et agissant sur l'élément de raccordement (36) transmettent le mouvement de translation du ten deur (10) sur la moitié avant de la pièce de jonction (21).
